# EUROPÄISCHE PATENTSCHRIFT

(11) **EP 2 419 036 B1**
(45) Veröffentlichungstag und Bekanntmachung des Hinweises auf die Patenterteilung: **07.11.2018**
(21) Anmeldenummer: 10711592.5
(22) Anmeldetag: 30.03.2010
(51) Int. Cl.: A61B 18/02

(54) **SYSTEM MIT KRYOCHIRURGISCHEM GERÄT, KRYOSONDE UND ADAPTER ZUR ÜBERDRUCKSICHERUNG**
SYSTEM HAVING CRYOSURGICAL DEVICE, CRYOPROBE, AND ADAPTER FOR OVERPRESSURE PROTECTION
SYSTÈME COMPRENANT APPAREIL CRYOCHIRURGICAL, CRYOSONDE ET ADAPTATEUR DE PROTECTION CONTRE LA SURPRESSION

(30) Priorität: 14.04.2009 DE 102009017370
(43) Veröffentlichungstag der Anmeldung: 22.02.2012
(73) Patentinhaber: Erbe Elektromedizin GmbH, 72072 Tübingen (DE)
(72) Erfinder: GROSS, Stefan, 72072 Tübingen (DE)
(74) Vertreter: Rüger Abel
(86) Internationale Anmeldenummer: PCT/EP2010/002017
(87) Internationale Veröffentlichungsnummer: WO 2010/118824

(56) Entgegenhaltungen:
- WO-A1-98/32383
- WO-A1-2009/022335
- WO-A2-2004/082749
- DE-A1-102006 003 571
- DE-U1- 9 002 065
- GB-A- 2 005 000
- US-A- 5 891 188
- US-A1- 2007 093 710
- US-A1- 2008 255 552

## Beschreibung

Die Erfindung betrifft ein System gemäß Anspruch 1. Das System umfasst einen Adapter zum Verbinden einer Versorgungseinrichtung mit einer Kryosonde, eine Kryosonde mit entsprechendem Adapter und ein kryochirurgisches Gerät.

Die Kryotherapie hat eine Vielzahl von Anwendungen. Kryosonden werden beispielsweise verwendet, um krankhaftes Gewebe zu zerstören, Gewebeproben zu entnehmen und/oder Fremdkörper zu entfernen. Des Weiteren kann die applizierte Kälte dazu verwendet werden, Blutungen zu stillen oder unvorteilhafte Gewebeveränderungen zu verhindern.

In der Kryotherapie, insbesondere in der Kryochirurgie, wird häufig Kälte mittels einer Sonde appliziert, um somit einen Heileffekt zu erzielen.

Um das entsprechende Instrument - also die Kryosonde - abzukühlen, gibt es verschiedene Verfahren. Häufig wird der Joule-Thomson-Effekt ausgenutzt. Dabei wird ein Fluid, insbesondere ein Gas, nahe dem Applikationsort über eine Düse in einer Expansionskammer expandiert, wodurch das Fluid eine Temperaturänderung erfährt. Die Kühlleistung basiert unter Anderem auf der Druckdifferenz, die am Ort der Expansion vorliegt. Um eine effektive Kühlung des Instruments zu gewährleisten, ist es notwendig, das expandierte Gas aus der Expansionskammer abzuleiten, ohne einen ungewollten Rückstau zu verursachen.

In der Praxis unterscheidet man Kryosonden mit Vordruckregelung und Hinterdruckregelung. Eine Kryosonde hat üblicherweise einen Zulauf und einen Rücklauf. Der Zulauf ist stets druckbeständig ausgebildet, wobei es Kryosonden gibt, deren Rücklauf nicht druckbeständig ist. Bei derartigen Kryosonden wird zur Einstellung der Kühlleistung der Kryosonde die Vordruckregelung verwendet. Hierbei stellt man den Druck im Zulauf je nach gewünschter Kühlleistung ein. Der Rücklauf hat einen im Wesentlichen atmosphärischen Druck. Bei der Hinterdruckregelung wird die Kühlleistung bei konstantem Druck im Zulauf über den Druck im Rücklauf geregelt.

Bei Sonden mit nicht druckfesten Rückläufen (Vordruckregelung) unterscheidet man zwischen offenen (offene Fluidkühlung) und geschlossenen Systemen. Bei den offenen Systemen wird das Fluid mittels Expansion mittelbar oder unmittelbar an die Umgebung abgegeben. Eine Rückführung innerhalb des Instruments erfolgt nicht oder nur bedingt. Das Fluid wird teilweise in das Organ entlassen oder von einem Endoskop aufgenommen. Hierbei gilt es jedoch zu berücksichtigen, dass zur Sicherheit des Personals gewährleistet werden muss, dass bei der Durchführung einer Operation nur geringe Mengen des Fluids in den Behandlungsraum entweichen.

Aus der DE 10 2006 003 571 A1 ist eine Versorgungseinrichtung für entsprechende Kryosonden mit einem Zulauf und einem Rücklauf bekannt, an die sowohl Kryosonden mit einer Vordruckregelung als auch mit einer Hinterdruckregelung angeschlossen werden können. Es ist wünschenswert, auch Kryosonden mit einem offenen System an eine derartige Versorgungseinrichtung anzuschließen. DE 90 02 065 U1 offenbart eine Vorrichtung zur Erzeugung eines Kaltgasstroms für die Kryotherapie. Auf einem Kältemittelgefäß sitzt ein Anschlusskopf, von dem sich ein Tragrohr nach unten zu einem Heizelement erstreckt. Am oberen Ende des Tragrohrs sitzt ein Phasenseparator.

WO 2004/082749 A2 behandelt eine Spritze zur Verabreichung therapeutischer Fluide. Das Dokument zeigt eine Injektionsnadel mit zwei angeschlossenen Spritzen, die von einem Motor über eine geeignete Spindel so betätigt werden, dass der Spritzeninhalt über die Injektionsnadel in das Gewebe gegeben wird.

GB 2 005 000 A beschreibt ein System zur kryomedizinischen Behandlung. Das System umfasst eine Leitung, an deren Ende ein Expansionsventil angeordnet ist, welche eine Kryosonde mit offener Fluidkühlung bildet. Die Sonde wird von einer Temperiereinrichtung gespeist, die zum Beispiel ein Wasserbehälter ist, in dem sich von unten gewärmtes Wasser befindet.

Des Weiteren ist es äußerst problematisch, dass es bei derartigen offenen Systemen keine Möglichkeit gibt, Verstopfungen des Zulaufs, eine Funktionsstörung der Druckeinstellvorrichtung und einen sich aufbauenden Überdruck im System zu erkennen. In derartigen Fällen kann es zu einer Beschädigung des Instruments, aber auch zu einer Gefährdung des Patienten oder des Anwenders kommen. Daher ist es wünschenswert, eine Überdruckabsicherung bzw. eine Überdrucksicherung auch für offene Kryosonden bzw. Kryosonden für eine offene Fluidkühlung bereitzustellen.

Es stellt sich also die Aufgabe, eine Überdruckabsicherung mit einer Kryosonde für eine offene Fluidkühlung bereitzustellen, wobei hohe Anforderungen an die Sicherheit sowie Effizienz des Systems gestellt werden. Des Weiteren sollte die Ausrüstung einer Kryosonde mit der Überdrucksicherung wirtschaftlich vertretbar sein und die Baugröße des Instruments nicht beeinflussen.

Diese Aufgabe wird erfindungsgemäß durch ein System gemäß Anspruch 1 gelöst.

Insbesondere wird die Aufgabe durch einen Adapter zum Verbinden einer Versorgungseinrichtung mit einer Kryosonde für eine offene Fluidkühlung gelöst, wobei der Adapter umfasst:
- einen ersten Fluidadapteranschluss zum Anschließen eines ersten Versorgungsanschlusses der Versorgungseinrichtung;
- einen zweiten Fluidadapteranschluss zum Anschließen eines zweiten Versorgungsanschlusses der Versorgungseinrichtung;
- einen Zulaufausgang zum Anschließen eines Zulaufs der Kryosonde;
- eine erste Fluidleitung zur fluiden Verbindung des ersten Fluidadapteranschlusses mit dem Zulaufausgang; und
- eine zweite Fluidleitung zur fluiden Verbindung des Zulaufausgangs mit dem zweiten Fluidadapteranschluss, wobei der Fluidfluss in der zweiten Fluidleitung gegenüber dem in der ersten Fluidleitung gedrosselt ist.

Ein zentraler Gedanke der vorliegenden Erfindung besteht also darin, vorhandene Versorgungsanschlüsse dafür zu nutzen, im Überdruckfall Fluid abzuleiten. Eine Versorgungseinrichtung für eine Kryosonde kann für geschlossene Systeme bereits entsprechende Sicherheitsmechanismen beinhalten. Durch den erfindungsgemäßen Adapter ist es möglich, diese Sicherheitsmechanismen zu nutzen. Um einen permanenten Abfluss des Fluids über die zweite Fluidleitung zu verhindern, muss der Fluidfluss in dieser Leitung gedrosselt werden. Dies kann innerhalb des Adapters oder in der Versorgungseinrichtung geschehen. Aufgrund des erfindungsgemäßen Adapters kann auf ein aufwändiges, groß bauendes und teures Überdruckventil in der Kryosonde verzichtet werden. Die zweite Fluidleitung sichert die angeschlossene Kryosonde effizient und zuverlässig ab.

Es ist möglich, mindestens eine Drossel zur Drosselung des Fluidflusses in der zweiten Fluidleitung vorzusehen. Somit sorgt der Adapter dafür, dass im Normalfall kein Fluid oder eine nur sehr geringe Menge über die zweite Fluidleitung abgeführt wird. Der wesentliche Teil des Fluids tritt über den Zulaufausgang in den Zulauf der Kryosonde ein und erbringt dort die gewollte Kühlleistung. Die Drossel kann gerätespezifisch ausgewählt sein. Alternativ kann die Drossel je nach angeschlossener Kryosonde eingestellt werden.

Der Adapter kann mindestens einen dritten Fluidanschluss zum Verschluss mindestens eines dritten Versorgungsanschlusses umfassen. Die Versorgungseinrichtung aus der DE 10 2006 003 571 A1 oder die aus der deutschen Anmeldung mit dem Aktenzeichen 10 2008 038 310 weisen mehrere Versorgungsanschlüsse, insbesondere drei, auf, um Kryosonden mit Vordruck- und Hinterdruckregelung zu betreiben. Es ist vorteilhaft, wenn der erfindungsgemäße Adapter nicht verwendete Versorgungsanschlüsse verschließt, so dass hier kein Fluid austritt. Dies erhöht die Sicherheit für den Anwender wie auch für den Patienten. Des Weiteren kann auf eine entsprechende Absicherung innerhalb der Versorgungseinrichtung verzichtet werden.

Mindestens einer der Fluidadapteranschlüsse kann zur Bildung einer Steckverbindung mit der Versorgungseinrichtung ausgebildet sein. Somit lässt sich der Adapter einfach und schnell an die Versorgungseinrichtung anschließen. Eine entsprechende Steckverbindung kann auch zum Anschließen der Kryosonde vorgesehen sein.

Die vorher genannte Aufgabe wird ebenfalls durch eine Kryosonde mit offener Fluidkühlung gemäß Anspruch 5 gelöst.

Eine derartige offene Kryosonde umfasst einen Adapter wie vorab beschrieben, sowie einen Fluidzulauf, wobei der Adapter eine Überdrucksicherung für den Fluidzulauf bereitstellt.

Der Adapter kann also mit der offenen Kryosonde verbindbar sein oder einen integralen Bestandteil dieser ausbilden. Die Kryosonde kann einen Sondenstecker zum Anschließen der Kryosonde an die Versorgungseinrichtung umfassen, wobei der Adapter ein, insbesondere integraler, Bestandteil des Sondensteckers ist.

Soweit der Adapter die mindestens eine Drossel in der zweiten Fluidleitung enthält, so kann diese kryosondenspezifisch ausgebildet sein. Hierdurch kann für eine bestimmte Kryosonde ein bestimmter Wert eingestellt werden, bei dem die Überdrucksicherung eingreift und das Fluid über den zweiten Fluidadapteranschluss ableitet.

Des Weiteren wird die Aufgabe durch ein kryochirurgisches Gerät gelöst, das umfasst: eine Versorgungseinrichtung zum Versorgen einer der vorab beschriebenen Kryosonden, wobei die Versorgungseinrichtung den ersten und den zweiten Fluidanschluss umfasst.

Die Versorgungseinrichtung kann also dazu genutzt werden, eine geeignete Überdrucksicherung bereitzustellen. Insbesondere werden vorhandene Mechanismen, die für geschlossene Systeme bereitgestellt werden, genutzt. Die zweite Fluidleitung kann in derartigen Versorgungseinrichtungen in den Versorgungsanschluss münden, der üblicherweise mit dem Rücklauf von geschlossenen Kryosonden verbunden wird.

Alternativ oder zusätzlich zu der mindestens einen Drossel in dem Adapter kann die Versorgungseinrichtung mindestens eine Drossel zum Drosseln des Fluidflusses durch den zweiten Versorgungsanschluss umfassen. Somit lässt sich der Fluidfluss in der zweiten Fluidleitung über die Versorgungseinrichtung regeln.

Die mindestens eine Drossel in der Versorgungseinrichtung kann zur Verhinderung eines Überdrucks in der Kryosonde einstellbar sein. Die Drossel sollte je nach angeschlossener Kryosonde eingestellt werden.

Die Versorgungseinrichtung kann eine Detektionseinrichtung zur Bestimmung des Typus der angeschlossenen Kryosonde und eine Steuereinheit umfassen, die mit der Detektionseinheit und der Drossel so in kommunikativer Verbindung steht, um die Drossel typenspezifisch einzustellen.

Das kryochirurgische Gerät, insbesondere die Versorgungseinrichtung, kann mindestens einen Sensor zur Bestimmung zumindest eines Fluidflusses und/oder eines Druckes durch den bzw. an dem zweiten Versorgungsanschluss umfassen. Diese Sensoren können genutzt werden, um den Druck im Zulauf der Kryosonde zu überwachen. Auf eine weitere Sensorik innerhalb der Kryosonde kann verzichtet werden.

Weitere vorteilhafte Ausführungsformen ergeben sich aus den Unteransprüchen.

Nachfolgend wird die Erfindung anhand von einigen Ausführungsbeispielen beschrieben, die mittels Abbildungen näher erläutert werden. Hierbei zeigen:
- - Fig. 1: ein kryochirurgisches Gerät mit einer offenen Kryosonde und einer angeschlossenen Versorgungseinrichtung;
- - Fig. 2: eine erste Ausführungsform des erfindungsgemäßen Adapters mit Versorgungseinrichtung; und
- - Fig. 3: eine zweite Ausführungsform des erfindungsgemäßen Adapters mit Versorgungseinrichtung.

In der nachfolgenden Beschreibung werden für gleiche und gleich wirkende Teile dieselben Bezugsziffern verwendet.

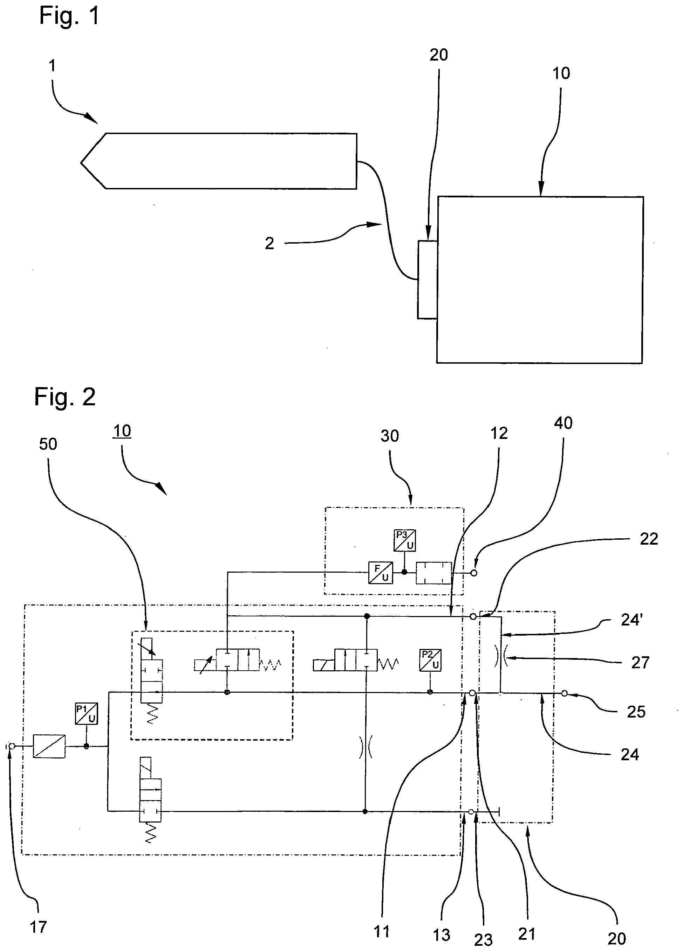

Fig. 1 zeigt ein erfindungsgemäßes kryochirurgisches Gerät, das eine Versorgungseinrichtung 10 und eine Kryosonde 1 umfasst. Zum Anschließen eines Kryosondenzulaufs 2 der Kryosonde 1 an die Versorgungseinrichtung 10 befindet sich in einer Steckverbindung der Kryosonde 1 ein Adapter 20. Bei der Kryosonde 1 handelt es sich um eine offene Kryosonde 1, die lediglich über den Kryosondenzulauf 2 verfügt. Ein Rücklauf ist innerhalb der Kryosonde 1 nicht vorgesehen. Das von der Versorgungseinrichtung 10 bereitgestellte Fluid wird im Normalfall nicht in die Versorgungseinrichtung 10 zurückgeführt, sondern nach dessen Expansion an die Umwelt abgegeben. Die Versorgungseinrichtung 10 ist dazu ausgebildet, weitere Kryosonden, beispielsweise solche mit einer Hinterdruckregelung zu betreiben. An der Versorgungseinrichtung 10 können des Weiteren geschlossene Kryosonden mit einer Vordruckregelung betrieben werden.

Fig. 2 zeigt den Adapter 20 und einen Ausschnitt der Versorgungseinrichtung 10 im Detail. Wie genauer in der deutschen Anmeldung mit dem Aktenzeichen 10 2008 038 310 beschrieben, lässt sich die Versorgungseinrichtung 10 in zwei sich unterscheidenden Betriebsmodi betreiben. In einem ersten Betriebsmodus wird die Kühlleistung der Kryosonde 1 über eine Vordruckregelung - also über das Einstellen des Drucks in dem Kryosondenzulauf 2 - eingestellt. In dem zweiten Betriebsmodus wird die Kühlleistung über den Rücklauf also durch eine Hinterdruckregelung eingestellt.

In dem ersten Betriebsmodus lassen sich offene und geschlossene Kryosonden betreiben. Erfindungsgemäß wird der Adapter 20 zum Betreiben der offenen Kryosonde 1 bereitgestellt. In dem besagten ersten Betriebsmodus wird das Fluid, das unter Druck in einer Fluidquelle 17 vorliegt, über eine Proportionalventileinheit 50 mit mindestens einem Proportionalventil zu einem ersten Versorgungsanschluss 11 geleitet. Dieser erste Versorgungsanschluss 11 steht in fluider Verbindung mit einem ersten Adapteranschluss 21 des Adapters 20. Von dem ersten Adapteranschluss 21 wird das Fluid über eine Adapterfluidleitung 24 zu einem Zulaufausgang 25 transportiert, der in den Kryosondenzulauf 2 mündet. Das Fluid wird innerhalb der Kryosonde 1, vorzugsweise nahe der Spitze, expandiert, kühlt die Kryosonde 1 und wird an die Umwelt abgegeben. Innerhalb des Adapters 20 besteht eine fluide Verbindung zwischen dem Zulaufausgang 25 und einem zweiten Adapteranschluss 22 in Form einer Überdruckleitung 24'. Der zweite Adapteranschluss 22 steht in fluider Verbindung mit einem zweiten Versorgungsanschluss 12, der mit einer Entlüftungseinheit 40 verbunden ist. Die Überdruckleitung 24' verfügt über eine Adapterdrossel 27, die den Fluidfluss über die Überdruckleitung 24' derart stark drosselt, dass im Normalbetrieb nur wenig Fluid über den zweiten Adapteranschluss 22 abgeleitet wird. Kommt es jedoch zu einem Überdruck in dem Kryosondenzulauf 2, so wird das Fluid sicher über die Adapterdrossel 27 zu dem zweiten Adapteranschluss 22 und dann zur Entlüftungseinheit 40 abgeleitet. Die Adapterdrossel 27 ist kryosondenspezifisch so eingestellt, dass eine Beschädigung der Kryosonde 1 oder eine Verletzung des Patienten oder des Anwenders vermieden wird. Erfindungsgemäß können weitere Adapter 20 bereitgestellt werden, um andere Kryosonden 1 zu betreiben.

Die Versorgungseinrichtung 10 umfasst des Weiteren einen dritten Versorgungsanschluss 13, der beispielsweise dazu verwendet werden kann, Kryosonden anzuschließen, die im zweiten Betriebsmodus betrieben werden. Der erfindungsgemäße Adapter 20 steht in fluider Verbindung mit diesem dritten Versorgungsanschluss 13. Er weist hierfür den dritten Adapteranschluss 23 auf, der den dritten Versorgungsanschluss 13 verschließt.

Die Versorgungseinrichtung 10 umfasst optional einen Flow-Sensor 30, der derart in fluider Verbindung mit dem zweiten Versorgungsanschluss 12 steht, dass die Flussgeschwindigkeit am zweiten Versorgungsanschluss 12 und/oder der dort anliegende Druck gemessen werden kann. Hiermit ist es möglich, eine Überdrucksituation frühzeitig zu erkennen und anzuzeigen. Des Weiteren kann die Versorgungseinrichtung 10 Gegenmaßnahmen ergreifen, um den auftretenden Druck abzubauen. Beispielsweise kann die Proportionalventileinheit 50 derart eingestellt werden, dass weniger Fluid mit geringerem Druck in die Kryosonde 1 eintritt.

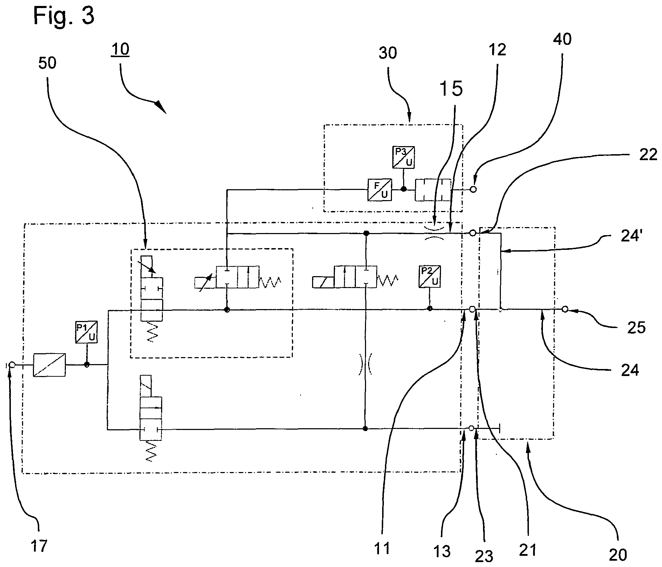

In einem weiteren Ausführungsbeispiel (vgl. Fig. 3) umfasst die Versorgungseinrichtung 10 eine Versorgungseinrichtungsdrossel 15. Diese ist unmittelbar nach dem zweiten Versorgungsanschluss 12 angeordnet und steht mit diesem in fluider Verbindung. Das über den zweiten Adapteranschluss 22 in die Versorgungseinrichtung 10 eintretende Fluid muss also über die Verbindungseinrichtungsdrossel 25 zu der Entlüftungseinheit 40 abfließen. Somit kann die Versorgungseinrichtungsdrossel 15 die Funktion der Adapterdrossel 27 übernehmen. Es ist also auch möglich, den erfindungsgemäßen Adapter 20 ohne eine entsprechende Adapterdrossel 27 bereitzustellen, wobei derselbe Effekt erzielt werden kann.

Wie in den Fig. 2 und 3 gezeigt, kann die Versorgungseinrichtung 10 zahlreiche Ventile und Sensoren umfassen, um einen geeigneten Druck an den einzelnen Versorgungsanschlüssen 11, 12, 13 bereitzustellen, oder ein ausreichendes Druckgefälle zwischen diesen Versorgungsanschlüssen 11, 12, 13 zu gewährleisten.

Wie bereits eingangs beschrieben, kann die Versorgungseinrichtung 10 auch dazu verwendet werden, andere Kryosonden z.B. mit einem Rücklauf zu betreiben. In dem ersten Betriebsmodus wird dann der Zulauf an dem ersten Versorgungsanschluss 11 und der Rücklauf an dem zweiten Versorgungsanschluss 12 angeschlossen. Im zweiten Betriebsmodus wird der Zulauf an dem dritten Versorgungsanschluss 13 und der druckfeste Rücklauf an dem ersten Versorgungsanschluss 11 angeschlossen. Somit kann bei entsprechender Konfiguration der Versorgungseinrichtung 10 der Druck im Rücklauf über die Proportionalventileinheit 50 geregelt oder gesteuert werden. Das zurückgeführte Gas wird jedesmal über die Entlüftungseinheit 40 abgeführt. Es ist leicht ersichtlich, dass aufgrund des Adapters 20 die einzelnen Komponenten der Versorgungseinrichtung 10 so verwendet werden können, dass auch eine Kryosonde 1 ohne Rücklauf effizient an dieser betrieben werden kann. Auf zusätzliche Bauelemente kann verzichtet werden. Somit können bei der Fertigung der Versorgungseinrichtung 10 und auch bei der Kryosonde 1 Kosten gespart werden.

### Bezugszeichenliste

- 1: Kryosonde
- 2: Kryosondenzulauf
- 10: Versorgungseinrichtung
- 11: erster Versorgungsanschluss
- 12: zweiter Versorgungsanschluss
- 13: dritter Versorgungsanschluss
- 15: Versorgungseinrichtungsdrossel
- 17: Fluidquelle
- 20: Adapter
- 21: erster Adapteranschluss
- 22: zweiter Adapteranschluss
- 23: dritter Adapteranschluss
- 24: Adapterfluidleitung
- 24': Überdruckleitung
- 25: Zulaufausgang
- 27: Adapterdrossel
- 30: Flow-Sensor
- 40: Entlüftungseinrichtung
- 50: Proportionalventileinheit

## Patentansprüche

1. System, bestehend aus:
einem Kryochirurgischen Gerät,
einer Kryosonde (1) mit offener Fluidkühlung wobei das Gerät eine Versorgungseinrichtung (10) zum Versorgen der Kryosonde aufweist und wobei die Versorgungseinrichtung (10) einen ersten und einen zweiten Versorgungsanschluss (11, 12) umfasst, sowie
einem Adapter (20) zum Verbinden der Versorgungseinrichtung (10) mit der Kryosonde (1), umfassend:
- einen ersten Fluidadapteranschluss (21) an den der erste Versorgungsanschluss (11) der Versorgungseinrichtung (10) angeschlossen ist;
- einen zweiten Fluidadapteranschluss (22) an den der zweite Versorgungsanschluss (12) der Versorgungseinrichtung (10) angeschlossen ist;
- einen Zulaufausgang (25) an den ein Zulauf (2) der Kryosonde (1) angeschlossen ist;
- eine erste Fluidleitung (24), über die der erste Fluidadapteranschluss (21) mit dem Zulaufausgang (25) in fluider Verbindung steht; und
- eine zweite Fluidleitung (24') über die der Zulaufausgang (25) mit dem zweiten Fluidadapteranschluss (22) in fluider Verbindung steht, wobei der Fluidfluss in der zweiten Fluidleitung (24') gegenüber dem in der ersten Fluidleitung (24) gedrosselt ist, so dass eine Überdrucksicherung für den Zulauf (2) der Kryosonde (1) bereitgestellt ist.

2. System nach Anspruch 1, **dadurch gekennzeichnet, dass** der Adapter mindestens eine Drossel (27) zur Drosselung des Fluidflusses in der zweiten Fluidleitung (24') aufweist.

3. System nach Anspruch 1 oder 2, **dadurch gekennzeichnet, dass** der Adapter mindestens einen dritten Fluidanschluss (23) zum Verschluss mindestens eines dritten Versorgungsanschlusses (13) der Versorgungseinrichtung (10) aufweist.

4. System nach einem der vorhergehenden Ansprüche, **dadurch gekennzeichnet, dass** mindestens einer der Fluidadapteranschlüsse (21 - 23) zur Bildung einer Steckverbindung mit der Versorgungseinrichtung (10) ausgebildet ist.

5. System nach Anspruch 1, **gekennzeichnet durch** einen Sondenstecker zum Anschließen der Kryosonde (1) an die Versorgungseinrichtung (10), wobei der Adapter (20) ein, insbesondere integraler, Bestandteil des Sondensteckers ist.

6. System nach Anspruch 1, **dadurch gekennzeichnet, dass**
- die Versorgungseinrichtung (10) mindestens eine Drossel (15) zum Drosseln des Fluidflusses durch den zweiten Versorgungsanschluss (12) umfasst.

7. System nach Anspruch 6, **dadurch gekennzeichnet, dass** die mindestens eine Drossel (15) zur Verhinderung eines Überdrucks in der Kryosonde (1) einstellbar ist.

8. System nach Anspruch 7, **dadurch gekennzeichnet, dass** die Versorgungseinrichtung (10) eine Detektionseinheit zur Bestimmung des Typus der angeschlossenen Kryosonde (1) und eine Steuereinheit umfasst, die mit der Detektionseinheit und der Drossel (15) in kommunikativer Verbindung steht, um die Drossel typenspezifisch einzustellen.

9. System nach einem der Ansprüche 1 bis 8, **gekennzeichnet durch**:
mindestens einen Sensor (30) zur Bestimmung zumindest eines Fluidflusses und/oder eines Druckes durch den bzw. an dem zweiten Versorgungsanschluss (12).

10. System nach einem der Ansprüche 1 bis 9, **gekennzeichnet durch**:
einen dritten Versorgungsanschluss (13) zum Anschluss einer Hinterdruckregelungs-Kryosonde.

## Claims

1. System comprising:
a cryosurgical device,
a cryoprobe (1) with open fluid cooling, wherein the device has a supply device (10) for supplying the cryoprobe, and wherein the supply device (10) has a first and a second supply connector (11, 12), and
an adapter (20) for connecting the supply device (10) to the cryoprobe (1),
comprising:
- a first fluid adapter connector (21) to which the first supply connector (11) of the supply device (10) is connected;
- a second fluid adapter connector (22) to which the second supply connector (12) of the supply device (10) is connected;
- an infeed output (25) to which an infeed (2) of the cryoprobe (1) is connected;
- a first fluid line (24) via which the first fluid adapter connector (21) is fluidically connected to the infeed output (25); and
- a second fluid line (24') via which the infeed output (25) is fluidically conencted to the second fluid adapter connector (22), wherein the fluid flow in the second fluid line (24') is choked relative to that of the first fluid line (24), so that an overpressure protection is provided for the infeed (2) of the cryoprobe (1).

2. System according to claim 1, **characterised in that** the adapter has at least one choke (27) for choking the fluid flow in the second fluid line (24').

3. System according to claim 1 or 2, **characterised in that** the adapter has at least one third fluid connector (23) for closing at least one third supply connector (13) of the supply device (10).

4. System according to any of the preceding claims, **characterised in that** at least one of the fluid adapter connectors (21 - 23) is configured to form a push-fit connection with the supply device (10).

5. System according to claim 1, **characterised by** a probe plug connector for connection of the cryoprobe (1) to the supply device (10), wherein the adapter (20) is an in particular integral part of the probe plug connector.

6. System according to claim 1, **characterised in that** the supply device (10) comprises at least one choke (15) for choking the fluid flow through the second supply connector (12).

7. System according to claim 6, **characterised in that** the at least one choke (15) is adjustable for preventing an overpressure in the cryoprobe (1).

8. System according to claim 7, **characterised in that** the supply device (10) comprises a detection unit for determining the type of cryoprobe (1) connected, and a control unit which is communicatively connected to the detection unit and to the choke (15) in order to adjust the choke type-specifically.

9. System according to any of claims 1 to 8, **characterised by** at least one sensor (30) for determining at least one fluid flow through and/or pressure at the second supply connector (12).

10. System according to any of claims 1 to 9, **characterised by** a third supply connector (13) for connection of a backpressure-regulator cryoprobe.

## Revendications

1. Système constitué :
d'un appareil cryochirurgical,
d'une cryosonde (1) à refroidissement par fluide ouvert, l'appareil présentant un dispositif d'alimentation (10) destiné à alimenter la cryosonde, et le dispositif d'alimentation (10) comportant un premier et un deuxième raccord d'alimentation (11, 12), ainsi que :
d'un adaptateur (20) destiné à relier le dispositif d'alimentation (10) à la cryosonde (1), comprenant :
- un premier raccord adaptateur de fluide (21) auquel est connecté le premier raccord d'alimentation (11) du dispositif d'alimentation (10) ;
- un deuxième raccord adaptateur de fluide (22) auquel est connecté le deuxième raccord d'alimentation (12) du dispositif d'alimentation (10) ;
- une sortie d'arrivée (25) à laquelle est raccordée une arrivée (2) de la cryosonde (1) ;
- une première et conduite de fluide (24) par l'intermédiaire de laquelle le premier raccord adaptateur de fluide (21) est en communication de fluide avec la sortie d'arrivée (25) ;
- une deuxième conduite de fluide (24') par l'intermédiaire de laquelle la sortie d'arrivée (25) est en communication de fluide avec le deuxième raccord adaptateur de fluide (22), le flux de fluide dans la deuxième conduite de fluide (24') étant réduit par rapport à celui dans la première conduite de fluide (24), de sorte qu'il existe une protection contre la surpression pour l'arrivée (2) de la cryosonde (1).

2. Système selon la revendication 1, **caractérisé en ce que** l'adaptateur présente au moins un élément d'étranglement (27) destiné à réduire le flux de fluide dans la deuxième conduite de fluide (24').

3. Système selon la revendication 1 ou 2, **caractérisé en ce que** l'adaptateur présente au moins un troisième raccord de fluide (23) destiné à obturer au moins un troisième raccord d'alimentation (13) du dispositif d'alimentation (10).

4. Système selon une des revendications précédentes, **caractérisé en ce qu'**au moins un des raccords adaptateurs de fluide (21 à 23) est conçu pour former une liaison enfichable avec le dispositif d'alimentation (10).

5. Système selon la revendication 1, **caractérisé en ce qu'**il comporte un connecteur de sonde destiné à raccorder la cryosonde (1) au dispositif d'alimentation (10), l'adaptateur (20) faisant partie, en particulier partie intégrante, du connecteur de sonde.

6. Système selon la revendication 1, **caractérisé en ce que** le dispositif d'alimentation (10) comprend au moins un élément d'étranglement (15) pour réduire le flux de fluide traversant le deuxième raccord d'alimentation (12).

7. Système selon la revendication 6, **caractérisé en ce que** l'élément d'étranglement (15), au nombre d'au moins un, peut être réglé afin d'empêcher une surpression dans la cryosonde (1).

8. Système selon la revendication 7, **caractérisé en ce que** le dispositif d'alimentation (10) comprend une unité de détection, destinée à déterminer le type de cryosonde (1) raccordée, ainsi qu'une unité de commande qui est en liaison de communication avec l'unité de détection et l'élément d'étranglement (15), afin de régler l'élément d'étranglement en fonction du type particulier.

9. Système selon une des revendications 1 à 8, **caractérisé en ce qu'**il comporte au moins un capteur (30) destiné à déterminer au moins un flux de fluide et/ou une pression passant par le deuxième raccord d'alimentation (12) ou appliqués à celui-ci.

10. Système selon une des revendications 1 à 9, **caractérisé en ce qu'**il comporte un troisième raccord d'alimentation (13) destiné au raccordement d'une cryosonde à régulation de pression secondaire.
